Europäisches Patentamt

European Patent Office

Office européen des brevets

(19)

(11) Veröffentlichungsnummer: **0 013 862**
**A1**

(12) ## EUROPÄISCHE PATENTANMELDUNG

(21) Anmeldenummer: **79810008.7**

(22) Anmeldetag: **26.01.79**

(51) Int. Cl.³: **A 61 B 17/18, A 61 F 1/00**

(43) Veröffentlichungstag der Anmeldung: **06.08.80**
**Patentblatt 80/16**

(84) Benannte Vertragsstaaten: **BE CH DE FR GB IT LU NL SE**

(71) Anmelder: **OSTEO AG, Bielstrasse 620, CH-2545 Selzach (CH)**

(72) Erfinder: **Mittelmeier, Heinz, Prof. Dr. med., An der Schutzhütte, D-Blieskastel (DE)**
Erfinder: **Moser, Heinz, Sparetweg 440, CH-2545 Selzach (CH)**
Erfinder: **Leu, Beat, Hintere Gasse 33, CH-2500 Biel (CH)**

(74) Vertreter: **Seehof, Michel et al, c/o AMMANN PATENTANWAELTE AG BERN Schwarztorstrasse 31, CH-3001 Bern (CH)**

(54) **Knochenplatte für die Osteosynthese.**

(57) Die Knochenplatte für die Osteosynthese besteht aus einem kohlefaserverstärkten Kunststoff, wobei die Kohlefasern (12) schichtweise angeordnet sind. Bei der dem Knochen zugewandten unteren Seite (13) besteht eine Schicht (14) von etwa 1 mm Dicke, die wenig oder gar keine Kohlefasern aufweist und dadurch einen mit dem Knochen vergleichbaren oder geringeren Elastizitätsmodul aufweist, während der Gehalt an Kohlefasern von Schicht zu Schicht zunimmt, um der Platte an der oberen Seite (15) einen Elastizitätsmodul zu verleihen, der mit demjenigen der herkömmlichen Metallplatten vergleichbar ist. Um die Platte leicht mit einem Heißluftstrom verformen zu können, wird ein thermoplastischer Kunststoff verwendet.

EP 0 013 862 A1

## Knochenplatte für die Osteosynthese

Die vorliegende Erfindung bezieht sich auf eine Knochenplatte für die Osteosynthese, insbesondere auf eine Osteosyntheseplatte mit Autokompressionseffekt. Die Osteosynthese, d. h. die operative Vereinigung von gebrochenen
oder durchtrennten Knochen mit schienenden oder stabilisierenden Hilfsmittel wird weltweit durchgeführt. Zur Osteosynthese dienen neben Marknägeln, Drähten, Schrauben,
auch insbesondere sogenannte Knochenplatten, die als interne Schiene unter Ueberbrückung des Bruch- oder Osteotomiespaltes am Knochen festgeschraubt werden. Die Osteosyntheseplatten werden vorwiegend aus rostfreiem Stahl,
teilweise auch aus gegossenen hochwertigen Metall-Legierungen (Kobalt-Chrom) hergestellt und können durch plastische Biegung der Knochenoberfläche in entsprechender Weise angepasst werden. Insbesondere kann man ihnen mit speziellen Biegegeräten auch eine sog. "Hohlbiegung" verleihen, welche nach dem Anschrauben zunächst zu einem Kontakt der kontralateralen und erst nach Kompression zu einem Kontakt der gesamten Fragmentfläche führt. Teilweise
werden diese Platten auch mit Vorspannung eingebracht, um
eine Kompression zwischen den Knochenbruchstücken zu erzielen, welche die Stabilität erhöht. Diese Vorspannung
wird teils mit separaten Spanngeräten und teils durch
Schraubenverschiebung und speziell gestalteten Plattenlöchern erreicht. Letztere Platten werden als selbst-

spannende Druckplatten bzw. Autokompressionsplatten bezeichnet und sind im Handel erhältlich.

Trotz Verwendung hochwertiger Metall-Legierungen besteht
jedoch bei den Metallplatten das nicht vollständig gelöste Problem der Kontaktkorrosion zwischen den Plattenlöchern und den daran anliegenden Schraubköpfen. Ein weiteres Problem entsteht durch den hohen Elastizitätsmodul,
der etwa 10-fach höher ist als der Elastizitätsmodul des
Knochens und unter der Platte zu einer biomechanisch bedingten Rarefizierung der Knochensubstanz führt. Nach
neueren Untersuchungen wird diese hauptsächlich dadurch bedingt, dass beim Festschrauben der Platte dieselbe mit
der Knochenunterlage eine so feste Reibungsverhaftung erfährt, dass der dortige Knochen eine dem Elastizitäts-
Modul der Platte entsprechende Einschränkung seiner elastischen Funktionen erfährt. Dadurch wird im Knochen der
physiologische Reiz zur Aufrechterhaltung seiner dichten
corticalen Knochenstruktur entzogen und es setzt eine Porosierung bzw. Spongiosierung dieses Knochenabschnittes ein. Diese Porosierung ist nachteilig, da der Knochen nach der
Fraktur bzw. Osteotomieheilung durch die Plattenentfernung
an dieser Stelle für längere Zeit geschwächt ist, was eine
Belastungseinschränkung erfordert und die Gefahr von Refrakturen beinhaltet, bis durch die zunehmende Funktionseinwirkung der Knochen sich wieder an die normalen Verhältnisse ohne Platte angepasst, d. h. die spongiosierte
Zone wieder verdichtet hat.

Es wurde bereits vorgeschlagen, durch Zwischenlagerung einer weichen Kunststoffschicht mit niedrigem Elastizitäts-
Modul den elastischen Funktionsentzug der Metallplatte im
Auflagegebiet zu vermindern und damit der Spongiosierung
entgegen zu wirken. Dieses Verfahren ist jedoch umständli-
und konnte daher keine praktische Anwendung finden. Ein weiterer Nachteil der Metallplatten ist die oft schwierige und teilweise hohe Kräfte erfordende Anpassung an
den Knochen, selbst bei Verwendung geeigneter Instrumente.

Es ist demgegenüber das Ziel der vorliegenden Erfindung,
eine Knochenplatte anzugeben, die einerseits keine
Korrosionsprobleme aufwirft , die insbesondere keine
Porosierung des Knochens im Bereich der Platte hervorruft und die sich leicht dem Knochen anpassen lässt.

Eine solche Knochenplatte zeichnet sich dadurch aus, dass
sie aus kohlefaserverstärktem Kunststoff besteht, wobei
die Kohlefasern derart angeordnet sind, dass die Platte
an der dem Knochen zugewandten, unteren Seite eine Schicht
mit einem demjenigen des Knochens vergleichbaren oder geringeren Elastizitätsmodul aufweist, und der Elastizitätsmodul der Oberseite der Platte demjenigen von rostfreiem
Stahl vergleichbar ist. In einer bevorzugten Ausführung
ist der Kunststoff ein mittels Heissluft verformbarer
thermoplastischer Kunststoff.

Die Erfindung wird nun im folgenden anhand zweier Ausführungsbeispiele näher erläutert, wobei

    Figur 1 eine Knochenplatte in Draufsicht,

    Figur 2 im Schnitt und im vergrösserten Massstab
    einen Teil der Platte von Figur 1 und

    Figur 3 einen Querschnitt, im vergrösserten Massstab, einer Ausführungsvariante zeigen.

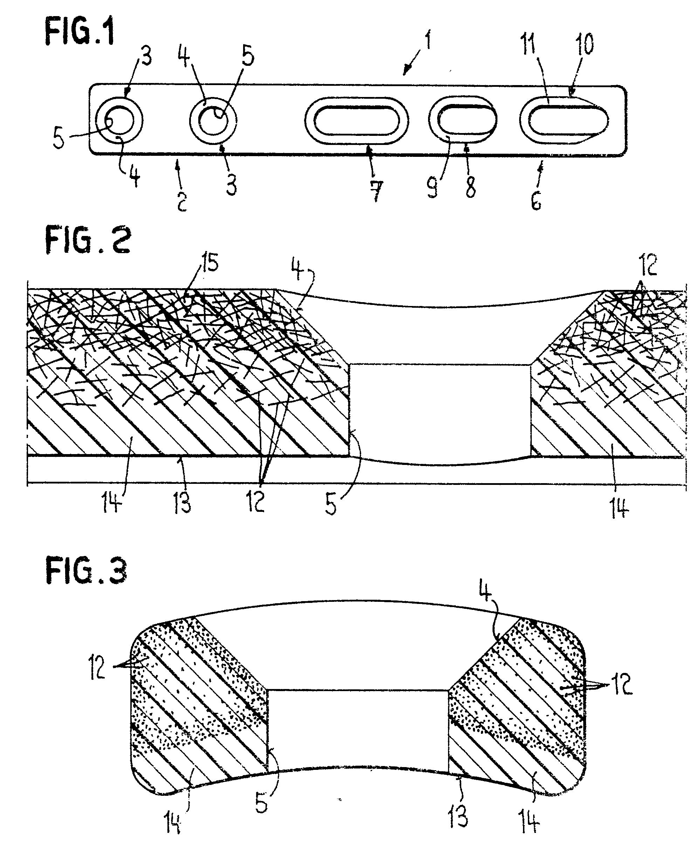

Man erkennt in Figur 1 eine schmale, selbstspannende
Knochenplatte 1, an der auf der Fixationsseite 2 zwei
Rundlöcher 3 angeordnet sind, mit einer Ansenkung 4, die der Kopfunterseite herkömmlicher Knochenschrauben sowohl mit konischen als auch mit halbkugeligen Schraubköpfen entspricht. Die Bohrung 5 dieser und der übrigen Löcher kann entweder für herkömmliche Knochenschrauben oder aber auch für dickere Spongiosaschrauben vorgesehen sein. Auf der Spannseite 6 der Platte befindet sich das Adaptationsgleitloch 7, welches sich in der Nähe der Fraktur bzw. Osteotomie befindet und die vorläufige Adaptation des zweiten Fragmentes an der Platte und eine exakte axiale Führung des zweiten Fragmentes, während des Kompressionsvorganges ermöglicht. Anschliessend an dieses Adaptationsgleitloch 7 befindet sich ein Druckloch 8, dessen Ansenkung 9 zur Plattenmitte hin unter 45$^\circ$ Neigung verläuft und das zuäusserst angebrachte Streckenspannloch 10, dessen Ansenkung 11 zur Plattenmitte hin eine Neigung von 27$^\circ$ aufweist. Die in diesen beiden letztgenannten Löchern exzentrisch, d. h. frakturfern eingebrachten Schrauben rutschen beim Eindrehen auf der schiefen Ansenkung in Richtung zur Fraktur bzw. Osteotomie hin und, da sie im Knochen stecken, bewirken sie unter Mitnahme des Knochens eine interfragmentäre Knochenkompression. Dabei verursacht das endständige Streckenspannloch 10 eine relativ grosse Verschiebestrecke bei guter Kompressionskraft und das Druckloch 8 eine optimale Transformation der Schraubkraft in eine längsgerichtete interfragmentäre Kompressionskraft zwischen den Knochenfragmenten. Die Anzahl und Anordnung der Löcher in Figur 1 entsprechen den kleinsten Knochenplatten. Bei längeren oder breiteren Knochenplatten erhöht sich entsprechend die Anzahl der Rundlöcher 3 und der Drucklöcher 8, wobei zwischen dem Adaptationsgleitloch 7 und den Drucklöchern 8 auch normale Rundlöcher angeordnet werden können, während in der Regel nur ein Streckenspannloch 10 und ein Adaptationsgleitloch 7 pro Platte verwendet werden.

Die Knochenplatte besteht gemäss Figur 2 aus einem geeigneten, thermoplastischen Kunststoff, der unter Einwirkung eines Heissluftstrahles verformbar sein muss. Dieser Kunststoff wird durch Kohlefasern 12 verstärkt, wobei die Fasern schichtweise angeordnet sind. An der Unterseite 13 der Platte, die auf den Knochen zu liegen kommt, befindet sich eine etwa 1 mm-dicke Schicht 14, die keine oder sehr wenig Kohlefasern aufweist, so dass der Elastizitätsmodul dieser unteren Schicht, je nach Wahl des Kunststoffes, etwa gleich oder weniger demjenigen des Knochens ist. Die darüberliegenden Schichten weisen einen immer grösseren Anteil an Kohlefasern auf, bis die oberste Schicht einen Elastizitätsmodul erreicht, der demjenigen der herkömmlichen rostfreien Knochenplatten entspricht.

In der Ausführungsvariante gemäss Figur 3 sind die Kohlefasern innerhalb der Schichten nicht regellos angeordnet, sondern im wesentlichen in der Längsrichtung ausgerichtet. Dabei befinden sich die Kohlefasern 12 vermehrt in den Randzonen, mit Ausnahme der untersten Schicht 14, die auch hier im wesentlichen kohlefasernfrei ist. Im Bereich der Ansenkungen, insbesondere der geneigten Ansenkungen, ist es zweckmässig, den Fasernanteil zu erhöhen und die Fasern zu ordnen, um eine bessere Druckübertragung zu gewährleisten. Dabei kann es zweckdienlich sein, die Fasern senkrecht zur Oberfläche der Ansenkung zu ordnen.

Einer der wesentlichsten Erfindungsgedanken, einen thermoplastischen Kunststoff mit schichtweise angeordneten Kohlefasern zu verwenden, ist nicht an die oben beschriebenen speziellen Ausführungsbeispiele gebunden. Es kann vielmehr eine Vielfalt von andern, zweckdienlich angeordneten Löchern, Gleitlöchern oder Drucklöchern vorgesehen werden und desweiteren eine Vielzahl von verschieden geformten Knochenplatten. So können auch Winkelplatten vorgesehen sein, bei denen eine Plattenhälfte in herkömmlicher Weise in ein epi-metaphysäres Knochenfragment, nach Vormeisseln, nagelartig eingeschlagen wird, weiter Platten, deren Fixationsseite t-förmig, löffelartig, schmetterlin.-
artig usw. gestaltet ist.

Um solche Platten aus thermoplastischem Kunststoff dem
Knochen anzupassen und die erforderliche Hohlbiegung zu verleihen, ist es genügend, diese in einem heissen Luftstrom
zu wärmen, wodurch sie leicht verformbar werden, um nach
dem Erkalten wieder die erforderliche Steifheit zu erlangen.

Platten, die vorgesehen sind, dauernd im Körper bzw. am
Knochen zu verbleiben, werden vorteilhafterweise mit
feinen Partikeln aus den mineralischen Komponenten
des natürlichen Knochengewebes, Apatit, oder aus Kalzium
und Phosphat bzw. enteiweisstem und entfettetem Knochenmaterial, beschichtet, um eine grössere Gewebeaffinität und
dauerhafte Integrierung der Platte in das Knochengefüge zu
erreichen. Diese Beschichtung fördert den Knochenwuchs an
diesen Stellen und bewirkt eine Verzahnung dieser Platte
am Knochen, wobei der Knochen entweder an diesen Mineralien
anwächst oder dieses Material allmählich eluiert und die
dort entstehenden Poren mit neu gebildetem, natürlichem
Knochengewebe ausgefüllt werden.

In einer weiteren Ausführungsvariante ist die Platte im
Bereich der Fraktur bzw. Osteotomie in der Dicke verstärkt und fällt zu den Enden hin ab, um vor allem im
Frakturbereich ausreichend Masse und Widerstandsmoment
zu haben. Ausserdem kann die Platte im Bereich der Löcher
seitlich überhöht sein, um den Verlust an Steifheit durch
die Lochbohrung auszugleichen.

0013862

- 1 -

Patentansprüche:

1. Knochenplatte für die Osteosynthese,
   dadurch gekennzeichnet,
   dass sie aus kohlefaserverstärktem Kunststoff besteht,
   wobei die Kohlefasern (12) derart angeordnet sind, dass
   die Platte (1) an der dem Knochen zugewandten, unteren
   Seite (13) eine Schicht (14) mit einem demjenigen des
   Knochens vergleichbaren oder geringeren Elastizitätsmodul aufweist, und der Elastizitätsmodul der Oberseite
   (15) der Platte mit demjenigen von rostfreiem Stahl vergleichbar ist.

2. Knochenplatte nach Anspruch 1,
   dadurch gekennzeichnet,
   dass der Kunststoff ein mittels Heissluft verformbarer
   thermoplastischer Kunststoff ist.

3. Knochenplatte nach Anspruch 1 oder 2,
   dadurch gekennzeichnet,
   dass sie selbstspannend ist und auf der Fixationsseite
   (2) Rundlöcher (3) und auf der Spannseite (6) ein Adaptationsgleitloch (7), ein Streckenspannloch (10) mit einer
   unter $27^\circ$ geneigten Ansenkung (11) und mindestens ein
   Druckloch (8) mit einer unter $45^\circ$ geneigten Ansenkung (9)
   aufweist.

4. Knochenplatte nach einem der Ansprüche 1 - 3,
   dadurch gekennzeichnet,
   dass die Kohlefasern (12) geordnet eingelagert sind, und
   insbesondere im Bereich der Ansenkungen derart, dass die
   von den Schraubenunterseiten ausgeübten Druckkräfte
   optimal auf die Platte übertragen werden.

5. Knochenplatte nach einem der Ansprüche 1 - 4,
   dadurch gekennzeichnet,
   dass die Bohrungen (5) der zwei äussersten Löcher (3)
   der Fixationsseite (2) jeweils für die Aufnahme von
   Spongiosaschrauben ausgebildet sind.

6. Knochenplatte nach einem der Ansprüche 1 - 4,
   dadurch gekennzeichnet,
   dass die Bohrungen sämtlicher Löcher (3, 8, 10) der
   Platte zur Aufnahme von Spongiosaschrauben ausgebildet sind.

7. Knochenplatte nach einem der Ansprüche 1 - 3,
   dadurch gekennzeichnet,
   dass mit Ausnahme der Unterschicht die Kohlefasern
   im wesentlichen in Längsrichtung und an den Aussenseiten eingelagert sind.

8. Platte nach einem der Ansprüche 1 - 7,
   dadurch gekennzeichnet,
   dass sie im Bereich zwischen Adaptationsgleitloch und
   nächstliegendem Rundloch eine grössere Dicke aufweist und
   zu den Enden hin abfällt.

9. Platte nach einem der Ansprüche 1 - 8,
   dadurch gekennzeichnet,
   dass sie im Bereich der Löcher seitlich überhöht ist.

0013862

10. Platte nach einem der Ansprüche 1 - 8,
    dadurch gekennzeichnet,
    dass sie mit das Anwachsen des Knochens fördernden
    Materialien, Apatit, Kalzium, Phosphat oder enteiweisstem und entfetteten Knochenmaterial, beschichtet
    ist.

0013862

1/1

**FIG.1**

**FIG.2**

**FIG.3**

**Europäisches Patentamt**

**EUROPÄISCHER RECHERCHENBERICHT**

0013862

Nummer der Anmeldung

EP 79 810 008.7

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>DE - A - 2 621 122</u> (SIGRI ELEKTRO-GRAPHIT) <br> * Seite 4, Absatz 2; Seite 5, Zeilen 13 bis 21; Seite 6, Absatz 2 * | 1,4,7 |
| | <u>DE - A - 2 049 111</u> (GULF ENERGY & ENVIRONMENTAL SYSTEMS) <br> * Ansprüche 1, 2, 4, 10; Seite 4, letzter Absatz bis Seite 6, Zeile 1; Fig. 6 * | 1 |
| | MEDIZINISCH-ORTHOPÄDISCHE TECHNIK, Band 94, Nr. 4, 1974, Stuttgart <br> H. MITTELMEIER "Prinzipien der Osteosynthese mit selbstspannenden Platten", Seiten 90 bis 99 <br> * Seite 96, Fig. 5, 6c; Seite 97, Fig. 7a * | 3,5,6 |
| | <u>DE - A - 2 621 123</u> (SIGRI ELEKTRO-GRAPHIT) <br> * Seite 6, Absatz 2 * | 4,7 |
| | <u>FR - A - 2 367 479</u> (E. DE BAZELAIRE et al.) <br> * Fig. 2 * <br> -- ./.. | 8 |

**KLASSIFIKATION DER ANMELDUNG (Int.Cl.²)**

A 61 B 17/18

A 61 F 1/00

**RECHERCHIERTE SACHGEBIETE (Int. Cl.²)**

A 61 B 17/18

A 61 F 1/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort <br> Berlin | Abschlußdatum der Recherche <br> 17-08-1979 | Prüfer <br> DROPMANN |
|---|---|---|

EPA form 1503.1 06.78

Europäisches
Patentamt

**EUROPÄISCHER RECHERCHENBERICHT**

0013862
Nummer der Anmeldung

EP 79 810 008.7
- Seite 2 -

| Kategorie | Kennzeichnung des Dokuments mit Angabe, soweit erforderlich, der maßgeblichen Teile | betrifft Anspruch |
|---|---|---|
| | US - A - 3 463 148 (H.T. TREACE) <br> * Fig. 1 * | 9 |
| | -- | |
| | DE - A - 2 708 917 (R. BOSCH) <br> * Anspruch 1; Seite 2 * | 10 |
| | ---- | |

**EINSCHLÄGIGE DOKUMENTE**

KLASSIFIKATION DER
ANMELDUNG (Int.Cl.²)

RECHERCHIERTE
SACHGEBIETE (Int. Cl.²)